# EUROPEAN PATENT SPECIFICATION

(11) **EP 3 996 720 B1**
(45) Date of publication and mention of the grant of the patent: **12.02.2025**
(21) Application number: 20838886.8
(22) Date of filing: 07.07.2020
(51) Int. Cl.: A61K 31/714, A61K 31/198, A61K 33/24, A61K 33/30, A61K 33/32, A61K 33/34, A61K 47/10, A23K 50/10, A23K 20/20, A23K 20/174

(54) **INJECTABLE NUTRITIONAL SUPPLEMENT**
INJIZIERBARE NAHRUNGSERGÄNZUNG
SUPPLÉMENT NUTRITIONNEL INJECTABLE

(30) Priority: 12.07.2019 AU 2019902471
(43) Date of publication of application: 18.05.2022
(73) Proprietor: Chemvet Australia Pty Ltd, Port Melbourne VIC 3207 (AU)
(72) Inventor: GRANT, Murray, Graham, Melbourne, Victoria 3207 (AU)
(74) Representative: Potter Clarkson
(86) International application number: PCT/AU2020/050710
(87) International publication number: WO 2021/007610

(56) References cited:
- EP-A1- 3 078 274
- WO-A1-2005/094842
- WO-A1-2005/094842
- WO-A1-2013/171538
- WO-A1-2013/171538
- GB-A- 822 127
- IE-B1- 58 361
- US-A- 2 939 821
- US-A1- 2015 328 318
- GORDON, J. L. ET AL.: "Effects of a combination butaphosphan and cyanocobalamin product and insulin on ketosis resolution and milk production", JOURNAL OF DAIRY SCIENCE, vol. 100, no. 4, 2017, pages 2954 - 2966, XP029958140, DOI: 10.3168/jds.2016-11925

## Description

### Field

The invention relates to an injectable nutritional supplement for livestock and in particular an aqueous injectable nutritional supplement for livestock containing vitamin B₁₂ and one or more trace elements, and to a method of providing a nutritional supplement to livestock.

### Background of Invention

Vitamin B₁₂ is a water-soluble vitamin which acts as a coenzyme for various metabolic functions, including fat and carbohydrate metabolism and protein synthesis. It is vital for glucose production in ruminants, which is essential to meet the high energy demands of growth and lactation. Vitamin B₁₂ deficiency results in reduced appetite, growth rates and milk production and can lead to anaemia and death depending on the extent of deficiency.

The supplementation of grazing livestock with vitamin B₁₂ and essential trace elements such as copper, cobalt, zinc and selenium has long been seen as a method of enhancing productivity particularly when these trace minerals are deficient or marginally deficient in their diets.

Grazing ruminants such as sheep, cattle, deer, goats, llamas etc. do not receive any vitamin B₁₂ directly from their diet. They rely on bacteria in the rumen to manufacture it for them. Vitamin B₁₂ manufactured in the rumen is then absorbed by the animal. Cobalt is a vital component of the vitamin B₁₂ molecule and is a trace mineral that is often deficient in the diet of grazing animals. When this occurs manufacture of vitamin B₁₂ by ruminal bacteria is compromised and the animal becomes vitamin B₁₂ deficient.

Cobalt administered by injection is not effective in correcting a vitamin B₁₂ deficiency caused by a cobalt deficiency because, when given this way, it does not find its way into the ruminal fluid allowing the ruminal bacteria to manufacture vitamin B₁₂ for the animal.

Nutritional supplements that provide a vitamin B₁₂ supplement together with one or more trace elements in a single administration are desirable particularly in countries which have pastures deficient in these minerals. Administration of supplements by subcutaneous injection is particularly convenient as injection is a convenient method to administer supplements especially to larger livestock where oral dosing is difficult.

Aqueous injectable concentrate compositions are desirable for tissue compatibility as well as for promoting the ready uptake of the trace minerals but vitamin B₁₂ is labile in aqueous compositions and degrades on storage resulting in a lower effective concentration than originally prepared.

Injectable formulations which combine vitamin B₁₂ and trace elements are particularly problematic as the trace mineral concentration, must be high so that the volume administered by injection is of an acceptable volume.

In the course of conducting experiments to prepare aqueous injectable compositions of vitamin B₁₂ and trace element complexes the present inventor found that the trace element complexes exacerbated the degradation problem of aqueous vitamin B₁₂ for which previously reported strategies for controlling degradation were not particularly effective. There is a need for a stable injectable composition of vitamin B₁₂ with one or more trace elements which is storage stable and convenient to administer.

IE 58361 B1 refers to a vitamin composition, particularly for veterinary use, containing minerals and trace elements, more especially intended for correcting or improving the feeding of ruminants. This document does not disclose the presence of EDTA or metal complexes thereof, nor trace elements at a concentration disclosed in the present application.

EP 3078274 relates to a trace element solution, which includes at least two metals selected from the group comprising selenium, copper, zinc, manganese, iron and chromium and which comprises a concentration of the metals of at least 60 mg/ml. At least one of the metals selected from the group comprising copper, zinc, manganese, iron and chromium may be provided in the form of an EDTA complex. This document does not disclose glycols or vitamin B₁₂ in the trace element solution.

WO 2005/094842 refers to compositions that include one or more vitamin B₁₂ compounds and one or more excipients that enhance solubility of the vitamin B₁₂ compounds. In aspects of the invention disclosed in WO 2005/094842, the excipients are alcohols, in particular ethanol, propylene glycol, a polyethylene glycol (PEG), glycerol, mannitol, sorbitol, Tween 20, or dimethylsulfoxide or a combination thereof, and/or a salt former. This document does not disclose the presence of trace elements or an EDTA complex thereof.

WO2013/171538 refers to a trace element solution, which comprises at least the following metals: zinc; manganese; selenium; and copper; and which comprises Vitamin B₁₂. The solution furthermore comprises butaphosphan to stabilize the Vitamin B₁₂ and the inclusion of butaphosphan may have synergistic activity with the minerals. This document does not disclose the presence of a glycol in the composition.

### Summary

Accordingly, there is provided an aqueous injectable nutritional supplement composition for livestock, comprising:
an EDTA complex of one or more trace elements;
vitamin B₁₂;
a water-soluble liquid selected from glycols, glycol ethers and mixtures thereof;

wherein the one or more trace elements are in an amount of 1 g to 100 g per litre of aqueous injectable composition, wherein the one or more trace elements comprise at least one of zinc, copper, manganese, selenium and chromium, wherein the amount of trace element is based on the element itself and not the whole trace element complex or mixture of complexes,
wherein the water-soluble liquid is selected from the group consisting of propylene glycol, glycol ethers of molecular weight no more than 300, polyethylene glycol of molecular weight no more than 300, dipropylene glycol monomethyl ether and mixtures thereof,
wherein the water-soluble liquid is present in the aqueous injectable composition in an amount of 20 g to 300 g per litre.

The more preferred water-soluble liquid is propylene glycol.

The one or more trace elements present in the composition typically include 10 g to 60 g of zinc per litre and 5 g to 30 g, preferably 10 g to 30 g, of copper per litre of aqueous injectable nutritional supplement composition. At least one of copper and zinc will typically be in the form of an EDTA complex. Preferably at least one and more preferably both zinc and copper are present as EDTA complexes.

In one embodiment the aqueous injectable nutritional supplement comprises:
1 g to 5 g of cyanocobalamin, hydroxocobalamin or mixture thereof per litre;
10 g to 60 g of zinc per litre;
5 g to 30 g, preferably 10 g to 30 g, of copper per litre;
Optionally, 1 g to 10 g selenium; and
50 g to 200 g per litre of water-soluble liquid selected from glycols, glycol ethers and mixtures thereof preferably 50 g to 200 g per litre propylene glycol per litre.

The aqueous injectable nutritional supplement composition comprises the trace elements in an amount of 1 g to 100 g per litre of aqueous injectable composition, preferably from 20 g to 100 g per litre of aqueous injectable composition.

In a further aspect there is provided a method of supplementing the nutritional status of livestock comprising administering by subcutaneous injection to the livestock (particularly ruminant livestock) an aqueous injectable nutritional supplement composition described herein.

### Detailed Description

We have found that the presence of a water-soluble liquid selected from glycols, glycol ethers and mixtures thereof has a very significant effect in stabilising vitamin B₁₂ against degradation in an aqueous solution in the presence of the EDTA complexes of trace elements. The presence of significant amounts of trace element complexes was found to exacerbate degradation of vitamin B₁₂ which could not be effectively controlled by methods normally used in formulation injectable vitamin B₁₂ compositions.

Strategies previously adopted for stabilising vitamin B₁₂ against degradation such as providing an acidic pH, the addition of aluminium sulfate or cysteine hydrochloride and formation of micelles were found to be ineffective in the presence of the high concentration of EDTA complexes of the trace elements.

The present inventor found that glycols and glycol ethers and in particular propylene glycol provided an unexpected stability of the vitamin B₁₂ in the presence of the even high concentration of trace element complexes.

The term "glycol" refers to any of a group of alcohols containing two hydroxyl groups, particularly including propylene glycol (PG) (propane 1,2-diol) andpropane-1,3-diol.

The term "glycol ether" refers to a compound in which one or both of the hydroxyl groups of a diol or condensate thereof are etherified. The term includes both monoglycol ethers and polyglycol ethers. Typically glycol ethers are of formula HO-[A--O]ₙ--R, wherein A represents ethylene or propylene moieties, R represents alkyl moieties of 1 to 4 carbon atoms, and n can assume values between 1 and 8, preferably between 1 and 4. Examples of glycol ethers include ethylene glycol monoisopropyl ether, ethylene glycol monobutyl ether, ethylene glycol monoisobutyl ether, ethylene glycol monoallyl ether, diethylene glycol monomethyl ether, diethylene glycol monoisopropyl ether, diethylene glycol monobutyl ether, diethylene glycol monoisobutyl ether, diethylene glycol monobenzyl ether, diethylene glycol dimethyl ether, diethylene glycol diethyl ether, diethylene glycol methyl ethyl ether, triethylene glycol monomethyl ether, triethylene glycol monobutyl ether, triethylene glycol dimethyl ether, polyethylene glycol monomethyl ether, propylene glycol monomethyl ether, propylene glycol monopropyl ether, dipropylene glycol monomethyl ether, tripropylene glycol monomethyl ether and 3-methoxy-3-methyl-1-butanol.

Preferred glycols and glycol ethers are water-soluble liquids, having molecular weight no more than 300, preferably no more than 200 such as no more than 150. The more preferred water-soluble liquids, selected from glycols and glycol ethers, are propylene glycol and polyethylene glycol of molecular weight no more than 300.

Throughout the description and the claims of this specification the word "comprise" and variations of the word, such as "comprising" and "comprises" is not intended to exclude other additives, components, integers or steps.

The aqueous injectable nutritional supplement comprises an EDTA complex of one or more trace elements. The one or more trace elements comprises at least one of zinc, copper, manganese, selenium and chromium. Preferably the trace elements comprise zinc, copper or mixture thereof. In one embodiment the trace elements comprise zinc, copper and selenium. The amount of the trace element referred to herein is based on the element itself and not the whole trace element complex or mixture of complexes.

The aqueous injectable nutritional supplement composition comprises the trace elements in an amount of 1 g to 100 g per litre of aqueous injectable composition. Preferred amounts of trace elements are in the range of from 20 g to 100 g such as 30 g/L to 100 g/L, 40 g/L to 100 g/L, 50 g/L to 100 g/L or 60 g/L to 100 g/L. Accordingly in one embodiment the trace elements comprise 1 g to 100 g per litre of zinc, copper or mixture thereof preferably 20 g to 100 g such as 30 g/L to 100 g/L, 40 g/L to 100 g/L, 50 g/L to 100 g/L or 60 g/L to 100 g/L. The total trace element content is typically up to 100 g/L of composition.

In one embodiment the content of 10 g to 60 g of zinc per litre, 5 g to 30 g of copper per litre such as 10 g to 30 g copper per litre or 10 g to 60 g of zinc per litre and 5 g to 30 g of copper per litre such as 10 g to 30 g copper per litre

The vitamin B₁₂ may be in a range of known forms. For example, the vitamin B₁₂ may comprise at least one of cyanocobalamin, hydroxocobalamin, hydroxocobalamin acetate, methylcobalamin and aquacobalamin and mixtures thereof. The amount of the vitamin B12 is typically 0.5 g to 8 g such as 1g to 8 g per litre of aqueous injectable composition or 1 g to 5 g per litre of aqueous injectable composition.

The water-soluble liquid selected from glycols, glycol ethers and mixtures thereof is typically present in the aqueous injectable composition in an amount of 20 g to 300 g per litre preferably 50 g to 250 g per litre, more preferably 80 to 250 g per litre such as 100 g/L to 250 g/L or 120 to 200 g/L of the aqueous injectable composition.

In one set of embodiments the aqueous injectable supplement composition comprises:
1 g to 5 g of cyanocobalamin, hydroxocobalamin or mixture thereof per litre;
10 g to 60 g of zinc per litre;
5 g to 30 g of copper per litre such as 10 g to 30 g;
1 g to 10 g selenium per litre; and
50 g to 200 g per litre of water-soluble liquid selected from glycols, glycol ethers and mixtures thereof, preferably 50 g to 200 g propylene glycol per litre.

The aqueous injectable nutritional supplement composition comprises an EDTA complex of one or more trace elements. The one or more EDTA complexes may be formed with any of a range of EDTA forms such as di-sodium EDTA, di-potassium EDTA, di-alkyl ammonium EDTA, di-ammonium EDTA or combination of two or more thereof. Typically, we have found it convenient to use the di-sodium EDTA in forming the trace element complexes.

Selenium deficiency in many pastures in countries such as Australia can severely inhibit growth of livestock and even result in death. The aqueous injectable nutritional supplement composition preferably comprises 1 g to 10 g selenium per litre of aqueous injectable composition such as 2 g to 8 g per litre.

In a further embodiment the aqueous injectable nutritional supplement composition comprises:
2 g to 4 g cobalamin or hydroxocobalamin acetate per litre
20 g to 45 g of zinc per litre;
10 g to 20 g copper per litre;
2 g to 10 g selenium per litre; and
80 g to 180 g per litre of water-soluble liquid selected from glycols, glycol ethers and mixtures thereof, preferably 50 g to 200 g propylene glycol per litre.

The pH of the aqueous injectable nutritional supplement composition is not narrowly critical and does not have a significant effect on the degradation of the vitamin B₁₂ in the presence of the trace element complexes. The pH is preferably the range of 5 to 8 such as 6 to 8, 6.5 to 7.5 or about 7.

In embodiments of the invention in which the pH control is to provide a tight range the composition may be buffered using a suitable buffer such as a citrate, phosphate, trimethamine (also called tris buffer) or acetate buffer. Phosphate buffers, such as sodium phosphate, potassium phosphate and mixtures thereof, are preferred.

The water content of the aqueous injectable nutritional supplement composition in one set of embodiments is 40% to 80% v/v

In a further embodiment the invention provides a method of supplementing the nutritional status of livestock comprising administering by subcutaneous injection to the livestock the aqueous injectable nutritional supplement composition as hereinbefore described.

The method is particularly useful in providing nutritional supplementation to ruminant animals especially bovine animals.

The invention will now be described with reference to the following examples. It is to be understood that the examples are provided by way of illustration of the invention and that they are in no way limiting to the scope of the invention.

### EXAMPLES

### Comparative Example 1

A formulation, the same as a purportedly stable, commercially available and registered solution of vitamin B₁₂ plus selenium was prepared.

The formulation contained the components in the amounts shown in Table 1.

**Table 1**

| Compound | Elemental g/L | g/L |
|---|---|---|
| Chlorocresol | | 1 |
| Sodium Acetate | | 1 |
| Sodium Chloride | | 2.67 |
| Hydroxocobalamin Acetate (Vit B₁₂) | | 1.8 |
| Cyanocobalamin (Vit B₁₂) | | 0.2 |
| Acetic Acid | | 1.09 |
| Sodium Selenate | 5.0 | 12 |
| Water to volume | | |

To the formulation of Table 1, water-soluble disodium Copper EDTA and disodium Zinc EDTA, were successfully added at the rate of 107g/L and 285 g/L respectively and then topped up with water to the required volume.

This provided a final formulation shown in Table 2.

**Table 2**

| Compound | Elemental g/L | g/L |
|---|---|---|
| Chlorocresol | | 1 |
| Sodium Acetate | | 1 |
| Sodium Chloride | | 2.67 |
| Hydroxocobalamin Acetate (Vit B₁₂) | | 1.8 |
| Cyanocobalamin (Vit B₁₂) | | 0.2 |
| Acetic Acid | | 1.09 |
| Sodium Selenate | 5.0 | 12 |
| Disodium Copper EDTA | 15 | 107 |
| Disodium Zinc EDTA | 40 | 285 |
| Selenium Selenate | 5 | 8 |
| Water to volume | | |

The formulation was fully dissolved with no evidence of precipitate.

This formulation was sent to Chemical Analysis, and analysed at 0 time and placed on stability storage at 5°C and 40°C (75%RH).

The results were as shown in Table 3.

**Table 3**

| Vitamin B₁₂ | T=0 (mg/mL) | | T=1 month (mg/mL) | |
|---|---|---|---|---|
| | 5°C | 40°C | 5°C | 40°C |
| Cyanocobalamin | 0.24 | 0.24 | 0.23 | 0.14 |
| Hydroxocobalamin Acetate | 0.49 | 0.49 | 0.26 | 0.03 |

Due to the obvious lack of stability of the formulation the trial was abandoned.

### Comparative Example 2

Formulations shown in Table 4 were prepared with single or combinations of chelated minerals (disodium EDTA) were formulated with Cyanocobalamin (CNCBL) and subjected to screening stability studies.

**Table 4**

| Formulatio n | Components (g/L) | | pH | 40°C Month 1 | 40°C Month 2 | 40°C Month 3 |
|---|---|---|---|---|---|---|
| 1 | CNCBL | 4.0 | 6.94 | CNCBL 3.3 | CNCBL 3.3 | |
| | Zn | 40 | | | | |
| | Water | to Vol | | | | |
| 2 | CNCBL | 4.0 | 4.4 | CNCBL 3.3 | CNCBL 3.3 | |
| | Cu | 16 | | | | |
| | Water | to Vol | | | | |
| 3 | CNCBL | 4.0 | 6.18 | CNCBL 3.1 | CNCBL 3.2 | |
| | Zn | 20.0 | | | | |
| | Cu | 7.5 | | | | |
| | Water | to Vol | | | | |
| 4 | CNCBL | 4.0 | 4.32 | CNCBL 3.3 | CNCBL 1.8 | |
| | Zn | 40 | | | | |
| | Water | to Vol | | | | |
| 5 | CNCBL | 2.5 | 5.9 | CNCBL 1.24 | | |
| | Zn | 40.0 | | | | |
| | Cu | 15.5 | | | | |
| | Water | to Vol | | | | |
| 6 | CNCBL | 2.5 | 5.9 | CNCBL 1.47 | | |
| | Zn | 40.0 | | | | |
| | Cu | 15.5 | | | | |
| | Se | 5.0 | | | | |
| | Water | to Vol | | | | |

| | | | | | | |
|---|---|---|---|---|---|---|
| CNBL - Cyanocobalamin | | | | | | |

The results shown in Table 4 demonstrate that in the presence of 40 g/L of Zinc (as disodium zinc EDTA), 15 g/L of Copper (as disodium copper EDTA) and 5.0 g/L of selenium (as selenium selenate) (in formulations 4, 5 and 6), which are the levels necessary to provide enough trace mineral in a single injection to livestock, sufficient levels of cyanocobalamin could not be maintained over time. Contrary to reported findings in the literature lowering the pH did not enhance the stability of the cyanocobalamin.

### Comparative Example 3

It has been claimed that the addition of Aluminium sulphate may enhance the stability of vitamin B₁₂ in aqueous solutions.

The solutions of Table 5 were formulated and subjected to accelerated stability testing at 40°C.

**Table 5**

| Formulation | Components (g/L) | | pH | 40°C Month 0 | 40°C Month 1 | 40°C Month 2 | 40°C Month 3 |
|---|---|---|---|---|---|---|---|
| 7 | CNCBL | 2.5 | 5.8 | CNCBL 2.0 | CNCBL 1.06 | | |
| | Zn | 40.0 | | | | | |
| | Cu | 15.5 | | | | | |
| | Se | 5.0 | | | | | |
| | Al. Sulphate | 62.5 | | | | | |
| 10 | CNCBL | 2.5 | 5.1 | CNCBL 2.3 | CNCBL 1.70 | CNCBL 1.36 | |
| | Zn | 40.0 | | | | | |
| | Cu | 15.5 | | | | | |
| | Se | 5.0 | | | | | |
| | Al. Sulphate | 10.0 | | | | | |

It was concluded that the addition of Aluminium sulphate did not enhance the stability of Cyanocobalamin in solutions containing high levels of trace minerals

### Comparative Example 4

Micelle formulations have been used to enhance the stability of molecules that are soluble in organic solvents but break down in aqueous solutions. Solutions including organic solvents singularly or in combination and a suitable surfactant were formulated and tested for stability of the cyanocobalamin in accordance with Table 6.

**Table 6**

| Formulation | Components (g/L) | | pH | 40°C Month 0 | 40°C Month 1 | 40°C Month 2 | 40°C Month 3 |
|---|---|---|---|---|---|---|---|
| 8 | CNCBL | 2.5 | 6.0 | CNCBL 2.31 | CNCBL 1.31 | CNCBL 1.09 | |
| | Zn | 40.0 | | | | | |
| | Cu | 15.5 | | | | | |
| | Se | 5.0 | | | | | |
| | G Formal* | 100.0 | | | | | |
| | ECT 80** | 10.0 | | | | | |

GFormal* - Glycerol Formal; ECT80 ** - Polyoxyethylene sorbitan monooleate (Tween 80); PG - propylene glycol.

It was concluded that these attempts to stabilise the vitamin B₁₂ in a micelle formulation were not successful.

### Example 1

In the process of combining organic solvents into the aqueous formulations of vitamin B₁₂ and high concentrations of trace minerals (during attempts to produce micelles containing the vitamin B₁₂) it was surprisingly discovered that by adding propylene glycol alone increased the stability of vitamin B₁₂ in a solution containing high amounts of trace minerals

The formulations and stability results are shown in Table 7.

**Table 7**

| Formulation | Components (g/L) | | 40°C Month 0 | 40°C Month 1 | 40°C Month 2 | 40°C Month 3 | 40°C Month 4 | 40°C Month 5 |
|---|---|---|---|---|---|---|---|---|
| 11 | CNCBL | 3.0 | CNCBL 3.12 | | CNCBL 2.36 | CNCBL 2.36 | | |
| | Zn | 30.0 | | | | | | |
| (Low mineral content) | Cu | 12 | | | | | | |
| | Se | 5.0 | | | | | | |
| | PG | 100 | | | | | | |
| | T80 | 2.0 | | | | | | |
| 12 | CNCBL | 3.0 | CNCBL 2.42 | | CNCBL 2.38 | CNCBL 2.33 | | |
| | Zn | 40.0 | | | | | | |
| | Cu | 15 | | | | | | |
| | Se | 5.0 | | | | | | |
| | PG | 100 | | | | | | |
| | T80 | 2.0 | | | | | | |
| 26 (23.4.18) | CNCBL | 2.50 | CNCBL 2.45 | | CNCBL 2.4 | | CNCBL 2.35 | CNCBL 2.40 |
| | Zn | 40.0 | | | | | | |
| | Cu | 20.0 | | | | | | |
| | Se | 5.0 | | | | | | |
| | PG | 150 | | | | | | |
| | | | | | | | | |
| 26 (23.6.18) | CNCBL | 2.50 | CNCBL 2.58 | CNCBL 2.3 | CNCBL 2.22 | CNCBL 2.15 | CNCBL 2.14 | |
| | Zn | 40.0 | | | | | | |
| | Cu | 20.0 | | | | | | |
| | Se | 5.0 | | | | | | |
| | PG | 150 | | | | | | |

The following Trial formulations were initially studied.
T80 - Polyoxyethylene sorbitan monooleate (Tween 80)

### Example 2

A further study mimicking commercial production and packaging was implemented and is reported below:
Method of manufacture:
**Part A**
   1. Load warm water (40°C) to mixing tank.
   2. Load disodium EDTA dihydrate powder to water and stir.
   3. Load Zinc Oxide powder to suspension and stir.
   4. Load Copper Hydroxide to suspension and stir.
   5. Continue stirring until all solids have dissolved; about 2 hours will be required.
   6. The reaction can be accelerated by heating the suspension at 40°C; a clear blue solution will be obtained.
   7. The pH of the solution at this stage should be 6.5 - 7.0.
   8. Load Sodium Selenate to batch and stir till dissolved.
**Part B**
   9. Load Propylene Glycol to small reactor.
   10. Load Cyanocobalamin powder to Propylene Glycol and stir till dissolved; slight warming to 40 deg. C will assist dissolution
   11.Load all of Part B to Part A in main tank and stir the solution till uniform.
   12. Measure the visible absorbance of the solution; 2% solution should have Absorbance at 550 nm > 0.31
   13. Filter the solution through 10 micron gauze to remove any insoluble solids, when transferring to holding tank.
   14. Filter product through 0.25 micron filter into sterile HDPE/LDPE "flow-pack" containers under aseptic conditions.

Once manufactured the formulation was placed in stability testing at 40°C. The formulation is shown in Table 8 and the stability testing results are shown in Table 9 below.

**Table 8**

| Formulation | Components (g/L) | |
|---|---|---|
| 26 (21.9.18) | CNCBL | 2.50 |
| | Zn | 40.0 |
| | Cu | 20.0 |
| | Se | 5.0 |
| | PG | 150 |

**Table 9**

| 40°C Month 0 | 40°C Month 1 | 40°C Month 2 | 40°C Month 3 | 40°C Month 4 | 40°C Month 5 | 40°C Month 6 | 40°C Month 7 |
|---|---|---|---|---|---|---|---|
| CNCBL 2.19 | CNCBL 2.12 | CNCBL 2.32 | CNCBL 2.31 | CNCBL 2.28 | CNCBL 2.32 | CNCBL 2.31 | CNCBL 2.25 |

### Example 3: Composition containing Dipropylene glycol monomethyl ether

A composition in accordance with the invention was prepared by combining premixed compositions identified as Part A and Part B below:
**Part A**

| | |
|---|---|
| Water | 65.0 grams |
| Na2H2EDTA | 32.5 grams |
| ZnO | 5.0 grams |
| Cu(OH)2 | 2.6 grams |
| Na Selenate | 1.2 grams |
| **Total** | **106.3 grams** |

**Part B**

| | |
|---|---|
| "DOWINOL" DPM | 14.0 grams |
| Propylene Glycol | 3.0 grams |
| CN-Cbl | 0.28 grams |
| **Total** | **17.3 grams** |

Note: DOWINOL DPM is dipropylene glycol monomethyl ether (DOWINOL is a trademark of the Dow Chemical Company).

Part B was added to Part A to provide a total combined batch weight of 123.6 grams.

The composition was subject to stability testing at 40°C for 100 days and the analytical results are shown in Table 10 below.

**Table 10**

| | 0 Days | 30 Days | 80 Days | 100 Days |
|---|---|---|---|---|
| SG (g/L) | 1200 | 1200 | 1200 | |
| pH | 6.16 | 6.36 | 6.47 | |
| Cu (g/L) | 14.8 | 15.3 | 15.4 | 15.6 |
| CN-Cbl (g/L | 2.33 | 2.41 | 2.4 | 2.45 |
| Zn (g/L) | 40 | (calculated) | | |
| Se (g/L | 5 | (calculated) | | |

The results show no loss of Vitamin B12 after 100 days at 40°C.

### Example 4: Composition containing Triethylene Glycol (PEG 150)

A composition in accordance with the invention was prepared by combining premixed compositions identified as Part A and Part B below:
**Part A**

| | |
|---|---|
| Water | 52.26 g |
| Na₂H₂EDTA | 27.4 g |
| ZnO | 5.1 g |
| Cu(OH)₂ | 1.0 g |
| Total | 87.06 g |

**Part B**

| | |
|---|---|
| Triethylene Glycol | 12.7 g |
| Cn- Cbl | 0.2 g |

Part B was added to Part A to provide a total combined batch weight of 100g.

The composition was subject to stability testing at 40°C and the analytical results are as shown in Table 11.

**Table 11**

| | 0 Days | 30 Days | 60 Days |
|---|---|---|---|
| SG (g/L) | 1235 | | |
| pH | 6.72 | 6.46 | |
| Cu (g/L) | 17.2 | 17.4 | 17.4 |
| CN-Cbl (g/L | 2.61 | 2.56 | 2.55 |
| Zn (g/L) | 40 | (calculated) | |
| Se (g/L | 5 | (calculated) | |

The results show virtually no loss of Vitamin B12 after 60 days at 40°C.

### Example 5: Composition containing Polyethylene Glycol MW 200

A composition in accordance with the invention was prepared by combining premixed compositions identified as Part A and Part B below:
**Part A**

| | |
|---|---|
| Water | 523.6 g |
| Na₂H₂EDTA | 274.0 g |
| ZnO | 41.0 g |
| Cu(OH)₂ | 22.0 g |
| Total | 870.6 g |

**Part B**

| | |
|---|---|
| PEG 200 | 127 g |
| Cn- Cbl | 2.4 g |
| Total | 129.4 g |

Part B was added to Part A to provide a total combined batch weight of 1000 g.

The composition was subject to stability testing at 40°C and the analytical results are as shown Table12.

**Table 12**

| | 0 Days | 30 Days | 60 Days |
|---|---|---|---|
| SG (g/L) | | | |
| pH | 6.21 | | |
| Cu (g/L) | 16.8 | 16.7 | 16.8 |
| CN-Cbl (g/L | 2.71 | 2.65 | 2.65 |
| Zn (g/L) | 40 | (calculated) | |
| Se (g/L | 5 | (calculated) | |

The results show Vitamin B12 stable after 60 days at 40°C.

### Example 6: Composition Manufactured under Commercial conditions in a commercial manufacturing facility

Some laboratory scale formulations may differ in performance and stability to larger scale formulations manufactured under commercial conditions in a commercial manufacturing plant

A composition in accordance with the invention in a commercial scale facility was manufactured by combining premixed compositions identified as Part A and Part B below. The composition was then filtered through a 0.2 µ filter and aseptically filled into 500mL HDPE/LDPE flow-pack containers
**Part A**

| | |
|---|---|
| Water | 7.7895 L |
| Na₂H₂EDTA | 4.1670 Kg |
| ZnO | 0.6150 Kg |
| Cu(OH)₂ | 0.3375 Kg |

**Part B**

| | |
|---|---|
| Polyethylene Glycol 200 | 1.7250 |
| Benzyl Alcohol | 0.18 Kg |
| Cn- Cbl | 0.0360 Kg |
| Total | 14.85 Kg |

After 3 months at ambient temperature the composition was subjected to storage at 40°C.

The analytical results are as shown in Table 13.

**Table 13**

| | Ambient | | 40°C | | | |
|---|---|---|---|---|---|---|
| | Month 0 | Month 3 | Month 4 | Month 5 | Month 6 | Month 7 |
| pH | 7.07 | 7.11 | 7.29 | 7.41 | 6.93 | |
| CnCbl | 2.06 | 1.99 | 1.97 | 1.95 | 2.03 | 2.02 |
| Copper | 16.3 | 16.2 | 16.5 | 16.2 | 16.5 | 16.3 |

The results show that a composition in accordance with the invention manufactured in a commercial scale facility under commercial conditions including sterilization by filtration and packaging remained stable with respect to Vitamin B12 for 7 months at 40°C.

### Example 7: Composition with the addition of a pH buffer

It may be advantageous to ensure that the pH of a composition in accordance with the invention was held between a specified narrow range. Accordingly, a phosphate/potassium buffer was added to the above composition between month 5 and month 6 in an amount calculated to hold the pH between pH 6.5 and pH 7.0.

The amount added was as follows:

| | |
|---|---|
| Disodium phosphate | 10.6 g |
| Monopotassium phosphate | 11.1 g |

### Analytical results were as shown in Table 14.

**Table 14**

| | Month 0 | Month 3 | Month 4 | Month 5 | Month 6 | Month 7 |
|---|---|---|---|---|---|---|
| pH | 7.07 | 7.11 | 7.29 | 7.41 | 6.93 | 6.85 |

The results showed that very small amount of buffer can stabilize the pH in a desired narrow range.

## Claims

1. An aqueous injectable nutritional supplement composition for livestock, comprising:
an EDTA complex of one or more trace elements;
vitamin B₁₂; and
a water-soluble liquid selected from glycols, glycol ethers and mixtures thereof,
wherein the one or more trace elements are in an amount of 1 g to 100 g per litre of aqueous injectable composition, wherein the one or more trace elements comprise at least one of zinc, copper, manganese, selenium and chromium, wherein the amount of trace element is based on the element itself and not the whole trace element complex or mixture of complexes,
wherein the water-soluble liquid is selected from the group consisting of propylene glycol, glycol ethers of molecular weight no more than 300, polyethylene glycol of molecular weight no more than 300, dipropylene glycol monomethyl ether and mixtures thereof,
wherein the water-soluble liquid is present in the aqueous injectable composition in an amount of 20 g to 300 g per litre.

2. The aqueous injectable nutritional supplement of claim 1, wherein the one or more trace elements comprise at least one of zinc and copper.

3. The aqueous injectable nutritional supplement of any one of the previous claims, wherein the trace elements are present in an amount of 20 g to 100 g per litre, preferably 20g to 100g per litre of zinc, copper or mixture thereof, preferably 10g to 60g of zinc per litre, 5g to 30g of copper per litre or 10g to 30g of copper per litre.

4. The aqueous injectable nutritional supplement composition of any one of the previous claims, wherein the trace elements comprise at least one further trace element selected from manganese, selenium and chromium.

5. The aqueous injectable nutritional supplement composition of any one of the previous claims, wherein the trace elements comprise zinc, copper and selenium, preferably 1 g to 10 g selenium per litre of aqueous injectable composition.

6. The aqueous injectable nutritional supplement composition of any one of the previous claims, wherein the vitamin B₁₂ comprises at least one of cyanocobalamin, hydroxocobalamin, hydroxocobalamin acetate, methylcobalamin and aquacobalamin and mixtures thereof in an amount of 0.5 g to 8 g per litre of aqueous injectable composition, preferably the amount of vitamin B₁₂ is an amount of 1 g to 8 g.

7. The aqueous injectable nutritional supplement composition of any one of the previous claims, comprising:
1 g to 5 g of cyanocobalamin, hydroxocobalamin or mixture thereof per litre;
10 to 60 g of zinc per litre;
10 g to 30 g of copper per litre; and
50 g to 200 g water-soluble liquid selected from glycols, glycol ethers and mixtures thereof per litre of aqueous injectable composition.

8. The aqueous injectable nutritional supplement composition of any one of the previous claims, wherein the EDTA complex is formed with di-sodium EDTA, di-potassium EDTA, di-alkyl ammonium EDTA, di-ammonium EDTA or mixture thereof.

9. The aqueous injectable nutritional supplement composition of any one of the previous claims, comprising:
2 g to 4 g cyanocobalamin or hydroxocobalamin acetate per litre
20 g to 45 g of zinc per litre;
10 g to 20 g copper per litre;
3 g to 10 g selenium per litre; and
80 to 180 g propylene glycol per litre.

10. The aqueous injectable nutritional supplement composition of any one of the previous claims, wherein the pH of the aqueous injectable composition is in the range of 5 to 8.

11. The aqueous injectable nutritional supplement composition of any one of the previous claims comprising a water content of 40% to 80% v/v.

12. A method of supplementing the nutritional status of livestock comprising administering by subcutaneous injection to the livestock an aqueous injectable nutritional supplement composition according to any one of the previous claims, preferably the livestock are ruminant animals.

13. The method according to claim 12, wherein the livestock are administered a dose of the aqueous injectable composition of between 2 ml to 20 ml.

## Patentansprüche

1. Wässrige injizierbare Nahrungsergänzungszusammensetzung für Vieh, umfassend:
einen EDTA-Komplex aus einem oder mehreren Spurenelementen;
Vitamin B₁₂; und
eine wasserlösliche Flüssigkeit, ausgewählt aus Glykolen, Glykolethern und Mischungen davon,
wobei das eine oder die mehreren Spurenelemente in einer Menge von 1 g bis 100 g pro Liter wässriger injizierbarer Zusammensetzung liegen, wobei das eine oder die mehreren Spurenelemente mindestens eines von Zink, Kupfer, Mangan, Selen und Chrom umfassen, wobei die Menge des Spurenelements auf dem Element selbst und nicht auf dem gesamten Spurenelementkomplex oder der Mischung von Komplexen basiert,
wobei die wasserlösliche Flüssigkeit aus der Gruppe ausgewählt wird, die aus Propylenglykol, Glykolethern mit einem Molekulargewicht von nicht mehr als 300, Polyethylenglykol mit einem Molekulargewicht von nicht mehr als 300, Dipropylenglykolmonomethylether und Mischungen davon besteht,
wobei die wasserlösliche Flüssigkeit in der wässrigen injizierbaren Zusammensetzung in einer Menge von 20 g bis 300 g pro Liter vorhanden ist.

2. Wässrige injizierbare Nahrungsergänzung nach Anspruch 1, wobei das eine oder mehrere Spurenelemente mindestens eines von Zink und Kupfer umfassen.

3. Wässrige injizierbare Nahrungsergänzung nach einem der vorstehenden Ansprüche, wobei die Spurenelemente in einer Menge von 20 g bis 100 g pro Liter, vorzugsweise 20 g bis 100 g pro Liter Zink, Kupfer oder einer Mischung davon, vorzugsweise 10 g bis 60 g Zink pro Liter, 5 g bis 30 g Kupfer pro Liter oder 10 g bis 30 g Kupfer pro Liter vorhanden sind.

4. Wässrige injizierbare Nahrungsergänzungszusammensetzung nach einem der vorstehenden Ansprüche, wobei die Spurenelemente mindestens ein weiteres Spurenelement umfassen, das aus Mangan, Selen und Chrom ausgewählt ist.

5. Wässrige injizierbare Nahrungsergänzungszusammensetzung nach einem der vorstehenden Ansprüche, wobei die Spurenelemente Zink, Kupfer und Selen umfassen, vorzugsweise 1 g bis 10 g Selen pro Liter wässriger injizierbarer Zusammensetzung.

6. Wässrige injizierbare Nahrungsergänzungszusammensetzung nach einem der vorstehenden Ansprüche, wobei das Vitamin B₁₂ mindestens eines von Cyanocobalamin, Hydroxocobalamin, Hydroxocobalaminacetat, Methylcobalamin und Aquacobalamin und Mischungen davon in einer Menge von 0,5 g bis 8 g pro Liter wässriger injizierbarer Zusammensetzung umfasst, wobei vorzugsweise die Menge an Vitamin B₁₂ eine Menge von 1 g bis 8 g ist.

7. Wässrige injizierbare Nahrungsergänzungszusammensetzung nach einem der vorstehenden Ansprüche, umfassend:
1 g bis 5 g Cyanocobalamin, Hydroxocobalamin oder einer Mischung davon pro Liter;
10 bis 60 g Zink pro Liter;
10 g bis 30 g Kupfer pro Liter; und
50 g bis 200 g wasserlösliche Flüssigkeit, ausgewählt aus Glykolen, Glykolethern und Mischungen davon, pro Liter wässriger injizierbarer Zusammensetzung.

8. Wässrige injizierbare Nahrungsergänzungszusammensetzung nach einem der vorstehenden Ansprüche, wobei der EDTA-Komplex mit Di-Natrium-EDTA, Di-Kalium-EDTA, Di-Alkylammonium-EDTA, Di-Ammonium-EDTA oder einer Mischung davon gebildet wird.

9. Wässrige injizierbare Nahrungsergänzungszusammensetzung nach einem der vorstehenden Ansprüche, umfassend:
2 g bis 4 g Cyanocobalamin oder Hydroxocobalaminacetat pro Liter
20 g bis 45 g Zink pro Liter;
10 g bis 20 g Kupfer pro Liter;
3 g bis 10 g Selen pro Liter; und
80 bis 180 g Propylenglykol pro Liter.

10. Wässrige injizierbare Nahrungsergänzungszusammensetzung nach einem der vorstehenden Ansprüche, wobei der pH-Wert der wässrigen injizierbaren Zusammensetzung im Bereich von 5 bis 8 liegt.

11. Wässrige injizierbare Nahrungsergänzungszusammensetzung nach einem der vorstehenden Ansprüche, umfassend einen Wasserinhalt von 40 % bis 80 % v/v.

12. Verfahren zum Ergänzen des Ernährungsstatus von Vieh, umfassend das Verabreichen einer wässrigen injizierbaren Nahrungsergänzungszusammensetzung durch subkutane Injektion an das Vieh nach einem der vorhergehenden Ansprüche, wobei das Vieh vorzugsweise Wiederkäuer sind.

13. Verfahren nach Anspruch 12, wobei dem Vieh eine Dosis der wässrigen injizierbaren Zusammensetzung zwischen 2 ml und 20 ml verabreicht wird.

## Revendications

1. Composition aqueuse de complément alimentaire injectable destinée au bétail, comprenant :
un complexe EDTA d'un ou plusieurs éléments traces ; de la vitamine B₁₂ ; et
un liquide hydrosoluble sélectionné parmi des glycols, des éthers de glycol et des mélanges de ceux-ci,
dans laquelle les un ou plusieurs éléments traces sont présents en une quantité de 1 g à 100 g par litre de la composition aqueuse injectable, dans laquelle les un ou plusieurs éléments traces comprennent au moins l'un parmi du zinc, du cuivre, du manganèse, du sélénium et du chrome, dans laquelle la quantité d'élément trace est basée sur l'élément lui-même et non l'ensemble du complexe d'éléments traces ou le mélange de complexes,
dans laquelle le liquide hydrosoluble est sélectionné dans le groupe consistant en le propylène glycol, les éthers de glycol de poids moléculaire inférieur ou égal à 300, le polyéthylène glycol de poids moléculaire inférieur ou égal à 300, l'éther monométhylique de dipropylène glycol et des mélanges de ceux-ci,
dans laquelle le liquide hydrosoluble est présent dans la composition aqueuse injectable en une quantité de 20 g à 300 g par litre.

2. Complément alimentaire aqueux injectable selon la revendication 1, dans lequel les un ou plusieurs éléments traces comprennent au moins l'un parmi du zinc et du cuivre.

3. Complément alimentaire aqueux injectable selon l'une quelconque des revendications précédentes, dans lequel les éléments traces sont présents en une quantité de 20 g à 100 g par litre, de préférence 20 g à 100 g par litre de zinc, de cuivre ou de mélange de ceux-ci, de préférence 10 g à 60 g de zinc par litre, 5 g à 30 g de cuivre par litre ou 10 g à 30 g de cuivre par litre.

4. Composition aqueuse de complément alimentaire injectable selon l'une quelconque des revendications précédentes, dans laquelle les éléments traces comprennent au moins un autre élément trace sélectionné parmi le manganèse, le sélénium et le chrome.

5. Composition aqueuse de complément alimentaire injectable selon l'une quelconque des revendications précédentes, dans laquelle les éléments traces comprennent du zinc, du cuivre et du sélénium, de préférence 1 g à 10 g de sélénium par litre de la composition aqueuse injectable.

6. Composition aqueuse de complément alimentaire injectable selon l'une quelconque des revendications précédentes, dans laquelle la vitamine B₁₂ comprend au moins un élément parmi de la cyanocobalamine, de l'hydroxocobalamine, de l'acétate d'hydroxocobalamine, de la méthylcobalamine et de l'aquacobalamine et des mélanges de ceux-ci en une quantité de 0,5 g à 8 g par litre de la composition aqueuse injectable, de préférence la quantité de vitamine B₁₂ est une quantité de 1 g à 8 g.

7. Composition aqueuse de complément alimentaire injectable selon l'une quelconque des revendications précédentes, comprenant :
1 g à 5 g de cyanocobalamine, d'hydroxocobalamine ou d'un mélange de celles-ci par litre ;
10 à 60 g de zinc par litre ;
10 g à 30 g de cuivre par litre ; et
50 g à 200 g d'un liquide hydrosoluble sélectionné parmi des glycols, des éthers de glycol et des mélanges de ceux-ci par litre de la composition aqueuse injectable.

8. Composition aqueuse de complément alimentaire injectable selon l'une quelconque des revendications précédentes, dans laquelle le complexe EDTA est formé avec de l'EDTA disodique, de l'EDTA dipotassique, de l'EDTA de dialkyl-ammonium, de l'EDTA de di-ammonium ou un mélange de ceux-ci.

9. Composition aqueuse de complément alimentaire injectable selon l'une quelconque des revendications précédentes, comprenant :
2 g à 4 g de cyanocobalamine, d'hydroxocobalamine ou d'un mélange de celles-ci par litre ;
20 g à 45 g de zinc par litre ;
10 g à 20 g de cuivre par litre ;
3 g à 10 g de sélénium par litre ; et
80 à 180 g de propylène glycol par litre.

10. Composition aqueuse de complément alimentaire injectable selon l'une quelconque des revendications précédentes, dans laquelle le pH de la composition aqueuse injectable se situe dans la plage de 5 à 8.

11. Composition aqueuse de complément alimentaire injectable selon l'une quelconque des revendications précédentes comprenant une teneur en eau de 40 % à 80 % v/v.

12. Procédé permettant de compléter l'état nutritionnel de bétail comprenant l'administration par injection sous-cutanée au bétail d'une composition aqueuse de complément alimentaire injectable selon l'une quelconque des revendications précédentes, de préférence le bétail correspond à des animaux ruminants.

13. Procédé selon la revendication 12, dans lequel est administrée au bétail une dose de la composition aqueuse injectable comprise entre 2 ml à 20 ml.
